# EUROPEAN PATENT SPECIFICATION

(11) **EP 1 355 687 B1**
(45) Date of publication and mention of the grant of the patent: **18.03.2009**
(21) Application number: 02709622.1
(22) Date of filing: 10.01.2002
(51) Int. Cl.: A61M 1/00, A61B 1/015

(54) **MODULAR ENDOSCOPE VALVE ASSEMBLY AND METHOD**
MODULARE ENDOSKOP-VENTILANORDNUNG UND METHODE
ENSEMBLE DE VALVE D'ENDOSCOPE MODULAIRE ET PROCEDE CONNEXE

(30) Priority: 17.01.2001 US 761784; 12.12.2001 US 22134
(43) Date of publication of application: 29.10.2003
(73) Proprietor: Innon Holdings, LLC, Valparaiso, IN 46383 (US)
(72) Inventor: DHINDSA, Avtar S., Valparsaiso, IN 46383 (US)
(74) Representative: McLeish, Nicholas Alistair Maxwell
(86) International application number: PCT/US2002/005126
(87) International publication number: WO 2002/056942

(56) References cited:
- US-A- 4 567 880
- US-A- 4 881 523
- US-A- 5 201 908
- US-A- 5 201 908
- US-A- 5 692 729

## Description

### BACKGROUND

The present invention relates to endoscopic surgical devices, and in particular to valve assemblies used to control the flow of irrigation fluid in such devices.

Endoscopic devices are customarily provided with an irrigation port that conducts an irrigation liquid to the viewing area at the end of the endoscopic device. One prior-art approach is to pressurize irrigation fluid in an IV fluid bag, and then to supply the pressurized irrigation fluid directly into an endoscope such as a ureteroscope. The endoscope includes integral valves that are generally operated with one hand while the other hand holds the handpiece of the endoscope. The advantage of this system is that the irrigation fluid is pressurized, thereby providing dilation of a ureter and good visibility, One potential disadvantage with this type of irrigating system is that it may be difficult to control fluid flow since two hands are required. If the fluid flow is not controlled properly, a stone can be dislodged back into the middle or upper ureter by an excessively high rate of flow. Also, in the event of extravasation, uncontrolled amounts of fluid can flow into the retroperitoneum.

Another type of irrigation system is a hand-operated, pressurized irrigating system commercially manufactured by Bard, Boston Scientific, and ACMI. This approach allows the amount of fluid being injected to be controlled, but the apparatus is relatively bulky. This system is mounted separately from the ureteroscope, and separate hands are used to hold the handpiece of the ureteroscope and to control the flow of irrigation fluid. On occasion, an assistant controls fluid flow while the physician holds the endoscope in the left hand and performs an endoscopic procedure with the right hand. In this case, precise control of the rate of fluid flow is difficult, because oral instructions are slower and less precise than direct manual control by the physician.

A third type of irrigation system includes two or more syringes that are operated by an assistant one at a time to supply pressurized irrigation fluid to the endoscopic device. Generally a valve is provided that allows the assistant to fill one of the syringes while the other is in use.

A fourth type of irrigation system includes a roller pump mechanism that delivers irrigation fluid at a constant set pressure. This system may incorporate a blow-off valve to prevent excessive pressure, and it is generally used in endoscopic specialties such as orthopedics in performing arthroscopies. This system requires the use of an electric motor and controller, and it is therefore costly and bulky.

Goodman U.S. Patent 4,567,880 discloses an endoscopic device having a three-way valve forming a permanent portion of the handpiece of the endoscope. This system allows a physician to control the flow of irrigation fluid with the same hand as that used to hold the handpiece. However, the Goodman system requires a specially constructed endoscope, and the irrigation system is an integral part of the endoscope. This limits the irrigation system to use with one particular endoscope.

US Patent 5,201,908 discloses a protective covering for a medical instrument, such as an endoscope. The covering includes an elongated hollow sheath having a wall of flexible material. The sheath is substantially gas and water impervious and includes a channel for the medical instrument. The sheath further includes auxiliary access channels associated with the sheath. The distal end of the protective covering is provided with a cap having an optically clear window to allow the lens portion of the medical instrument to operate.

The present invention is directed to an improved system and method for controlling the flow of irrigation fluid in an endoscopic device.

### SUMMARY

In accordance with the present invention there are provided a modular endoscope valve assembly and a method for enhancing control efficiency of a medical endoscope as recited in the accompanying claims.

The preferred embodiment described below includes a modular valve assembly having a housing that carries an inlet port, an outlet port and a valve. The valve can be manually controlled by a user with the hand holding the endoscope to selectively allow or block fluid flow from the inlet port to the outlet port.

In use, the housing is releasably mounted to the handpiece of an endoscope by a strap, or a strap in combination with a pressure-sensitive adhesive. The inlet port is connected to a source of pressurized irrigation fluid and the outlet port is connected to the irrigation port of the endoscope. The physician can then use a single hand to perform both the function of holding the handpiece and the function of controlling the flow of irrigation fluid. Typically, the physician holds the handpiece in the palm, using the thumb and fingers of one hand. The physician controls the flow of irrigation fluid with one finger of the hand that is holding the handpiece. This leaves the other hand free for performing a surgical procedure via the working port of the endoscope, e.g., positioning and manipulating a stone extraction basket. Once the surgical procedure is completed, the modular housing can simply be removed from the endoscope and discarded. This eliminates the need to clean the valve or the ports of the valve assembly.

The housing may be formed in one or more parts, and it may include a second valve to allow the physician to control the application of suction in addition to the flow of irrigation fluid.

This section has been provided by way of general introduction, and it should not be used to narrow the scope of the following claims.

### BRIEF DESCRIPTION OF THE DRAWINGS

Figure 1 is a perspective view of a ureteroscope on which is mounted a modular valve assembly.
Figure 2 is a top perspective view of the valve assembly of Figure 1, prior to mounting on the ureteroscope.
Figure 3 is a bottom perspective view of the valve assembly of Figure 2.
Figure 4 is a perspective view of the valve assembly of Figures 2 and 3 connected to a source of pressurized irrigation fluid.
Figures 5 and 6 are schematic views showing the valve of the valve assembly of Figures 1-3 in the opened and closed positions, respectively.
Figures 7 and 8 are schematic views of an alternative, rotary-motion valve in the opened and closed positions, respectively.
Figure 9 is a fragmentary sectional view of another modular valve assembly mounted on a ureteroscope.
Figure 10 is a fragmentary sectional view of yet another modular valve assembly.
Figure 11 is a fragmentary sectional view of the ureteroscope of Figure 10 and a cover plate.
Figure 12 is a fragmentary sectional view of another modular valve assembly mounted on a ureteroscope.
Figure 13 is a cross-sectional view of another modular valve assembly of this invention including a mechanical latch to hold the valve in a selected position.
Figures 14, 15 and 15 are three sectional views of another modular valve assembly of this invention in three different positions.
Figure 17 is a sectional view of another modular valve assembly of this invention.
Figures 18, 19 and 20 are side views of three additional modular valve assemblies of this invention.

### DETAILED DESCRIPTION OF THE PRESENTLY PREFERRED EMBODIMENTS

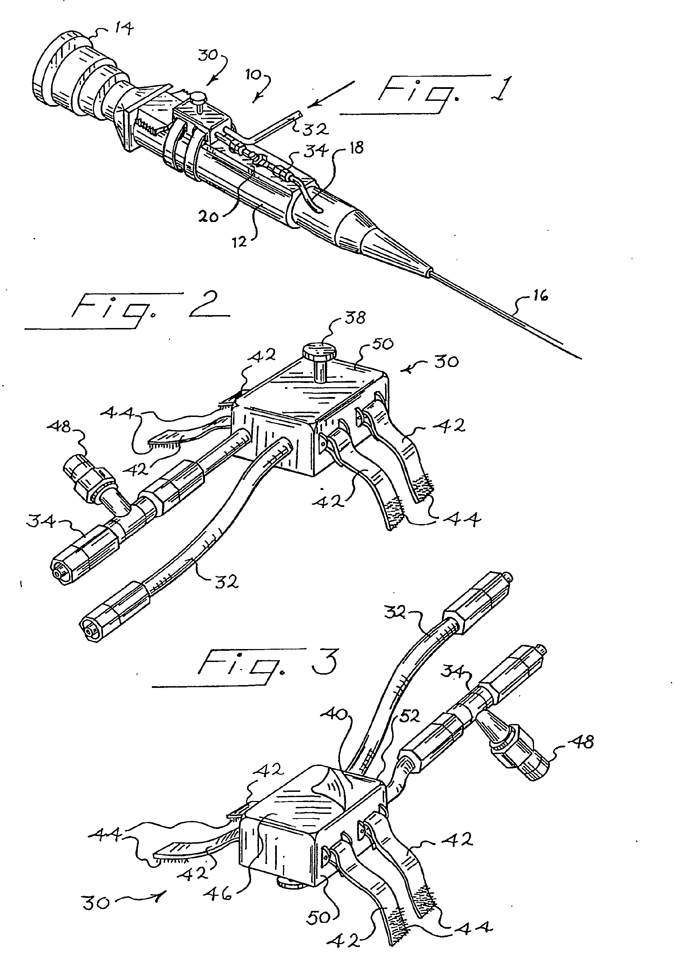

Turning now to the drawings, Figure 1 is a perspective view of an endoscopic device 10 that in this embodiment is a ureteroscope. The ureteroscope 10 includes a handpiece 12 that carries an eyepiece 14 at one end and a shaft 16 at the other end. An irrigation port 18 is carried by the handpiece 12, and irrigation fluid introduced via the irrigation port 18 is conducted to the viewing area at the end of the shaft 16 that is inserted into the patient. The handpiece 12 also defines an exterior surface 20.

The endoscopic device 10 can take any suitable form, and the present invention is not limited to any particular embodiment. For example, the endoscopes of any of the following U.S. Patents can be adapted for use with this invention: Wallace U.S. Patent 2,691,370, Ibe U.S. Patent 4,132,227, Goodman U.S. Patent 4,567,880, Cho U.S. Patent 5,083,549, Muller U.S. Patent 5,199,417, Bonati U.S. Patent 5,290,279, and Odanacka U.S. Patent 5,830,126. Conventional endoscopes such as the ureteroscopes manufactured by ACMI, Wolf, Olympus and Storz are also well-adapted for use with this invention. This list is intended only by way of illustration, in the widest variety of ureteroscopes, arthroscopes, laparoscopes, hysteroscopes, sinuscopes, and endoscopes adapted for other specialties can be used with this invention, including flexible, semi-rigid, and rigid endoscopes.

In use, the physician holds the handpiece with one hand, thereby presenting the eyepiece for viewing and positioning the shaft as desired. The other hand is typically used to manipulate surgical tools introduced into the patient via the working port on the shaft. As shown in Figure 1, a modular endoscope valve assembly 30 is releasably secured to the handpiece 12. This valve assembly 30 is shown in greater detail in Figures 2 and 3, and it includes an inlet port 32 and an outlet port 34. In use the inlet port 32 is releasably connected to a source of pressurized irrigation fluid, and the outlet port 34 is releasably connected to the irrigation port 18 of the handpiece.

The valve assembly 30 includes a valve that is interposed between the inlet port 32 and the outlet port 34 and is controlled by a valve actuator 38. The valve assembly 30 also includes a housing 50 that includes a mounting surface 52. The mounting surface 52 carries a pressure-sensitive adhesive 40 initially covered by a release paper 46. The housing 50 also supports a pair of straps 42 that include respective hook-and-loop fasteners 44. A contrast-introduction port 48 is provided in fluid communication with the outlet port 34. Check valves, not shown, can be provided to prevent flow from the outlet port 34 to the contrast-introduction port 48 and vice-versa.

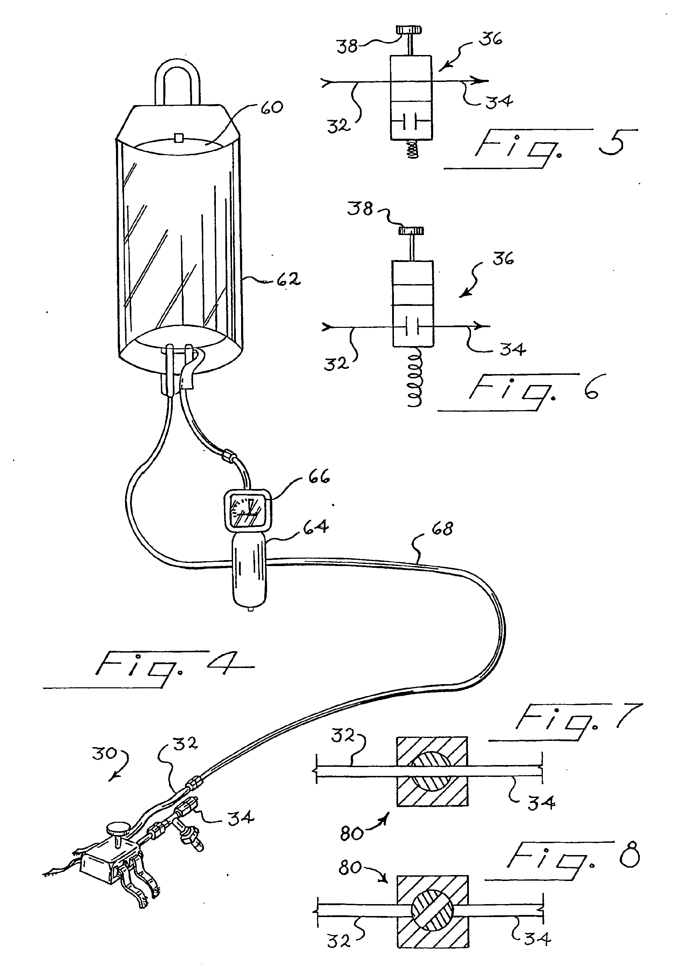

Figure 4 shows the manner in which the inlet port 32 of the valve assembly 30 can be releasably connected to a source of pressurized irrigation fluid, in this case contained within an IV bag 60. The IV bag 60 is disposed within a pressure cuff 62 that can be inflated with an inflator 64 to a pressure indicated by a pressure gauge 66. Standard Luer-lock fittings can be used to connect the inlet port 32 to a tube 68 that is in turn connected to the IV bag 60. The IV bag contains a conventional irrigation fluid, which is pressurized by inflating the pressure cuff 62 to a desired pressure with the inflator 64.

Figures 5 and 6 show two schematic views of the valve 36 of the valve assembly 30. In Figure 5 the valve actuator 38 is depressed and the valve 36 allows fluid flow from the inlet port 32 to the outlet port 34. When manual pressure is removed from the valve actuator 38, the valve 36 returns to the position of Figure 6, in which the valve 36 blocks the flow of fluid between the inlet and the outlet ports 32, 34. Alternatively, the valve 38 may be arranged such that fluid flow is blocked when the actuator 38 is depressed and unblocked when the actuator 38 is released.

The valve 36 of Figures 5 and 6 is a linear valve that slides along a linear axis between the opened position of Figure 5 and the closed position of Figure 6. Other types of valves are suitable, including the linear valve of U.S. Patent 4,238,108 and the rotary valve 80 of Figures 7 and 8. A rotary valve 80 rotates about an axis between the opened position of Figure 7 and the closed position of Figure 8, and the associated valve actuator (not shown in Figures 7 and 8) moves in a rotary motion as well.

In use, the valve assembly 30 is distributed separately from the endoscope 10. In this embodiment, the valve assembly 30 is shaped to fit on a wide variety of endscopes 10 such that the endoscope 10 does not have to be specially shaped or configured for the valve assembly 30. Prior to an endoscopic procedure, the release paper 46 is removed, thereby exposing the pressure-sensitive adhesive 40 on the mounting surface 52. Then the valve assembly 30 is placed on the exterior surface 20 of the endoscope 10, and the pressure-sensitive adhesive 40 releasably holds the valve assembly 30 in place. The straps 42 are positioned around the handpiece 12, and the hook-and-loop fasteners 44 are secured together to hold the valve assembly 30 in place.

Either before or after the valve assembly 30 is secured to the handpiece 12, the inlet port 32 is releasably secured to the tube 68 (Figure 4) and the outlet port 34 is releasably secured to the irrigation port 18 of the handpiece 12 (Figure 1). Preferably, the valve assembly 30 is flushed after it is connected to the tube 68 and before it is connected to the irrigation port 18.

The physician then performs the desired endoscopic procedure, using a single hand both to hold the handpiece 12 and to control the flow of pressurized irrigation fluid with the valve assembly 30. A part of the hand that holds the handpiece (e.g. the fingers or the heel) is used to move the valve actuator.

Once the endoscopic procedure has been completed, the valve assembly 30 can simply be removed from the endoscope 10 by releasing the hook-and-loop fasteners 44 and lifting or twisting the valve assembly 10 away from the handpiece 12 until the pressure-sensitive adhesive 40 releases.

The valve assembly 30 described above uses both a pressure-sensitive adhesive and a set of straps to releasably secure the valve assembly 30 in place on the handpiece 12. In alternative arrangements the adhesive may be used without the reinforcing straps. In one embodiment, the reinforcing straps can be used without the adhesive. The strap may be varied widely. For example, the strap may pass over the top of the valve assembly, and the actuator may pass through an opening in the strap. The strap may be fixed to the valve assembly or not. Also, other types of fasteners can be used to releasably hold the valve assembly in place on the endoscope.

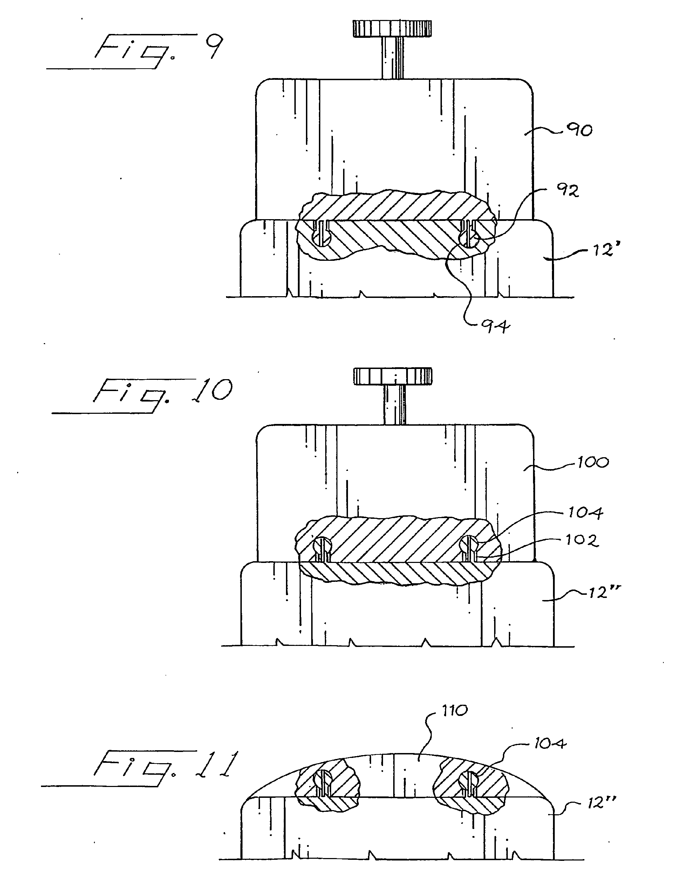

Figure 9 shows an alternative arrangement of a modular valve assembly. The valve assembly 90 is identical to the valve assembly 30 described above except for the manner of releasably attaching the valve assembly 90 to the handpiece 12'. In this case the valve assembly 90 is provided with mechanical fasteners 92 and the handpiece 12' is provided with mating mechanical fasteners 94 such that the valve assembly 90 can be snapped in place on the handpiece 12' and removed from the handpiece 12' as desired. In this example, the fasteners 92 take the form of protruding studs and the mating fasteners 94 take the form of recesses shaped to receive the fasteners 92 in a snap-lock action.

Figure 10 shows portions of a third valve assembly 100 which is similar to that of Figure 9 except that the fasteners 102 are shaped as recesses and the mating fasteners 104 are shaped as protruding studs that fit into the fasteners 102 in a snap-lock manner.

Figure 11 shows the handpiece 12" of Figure 10 with a cover 110 snapped in place on the mating fasteners 104. The cover 110 covers the mating fasteners 104 when a valve assembly is not in place on the handpiece 12".

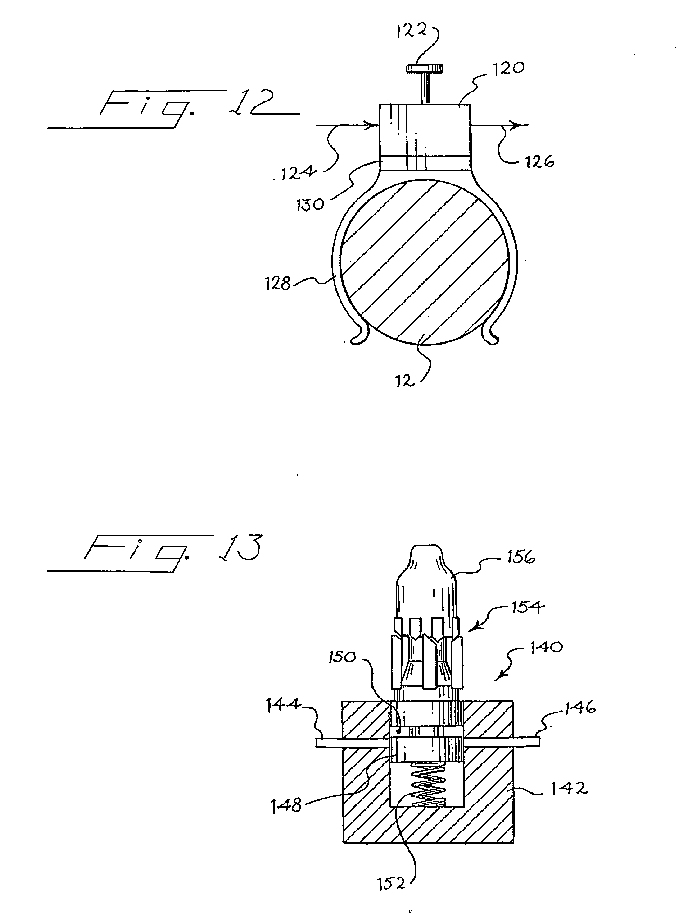

Figure 12 shows another modular valve assembly 120 mounted in place on the handpiece 12 of an endoscopic device. The valve assembly 120 includes an actuator 122, an inlet port 124, and an outlet port 126. The valve assembly 120 is mounted on a base 130, and the base 130 supports a spring clip 128 that is designed to fit at least partially around the handpiece 12 and to releasably hold the base 130 and therefore the valve assembly 120 in position on the hand-piece 12. The spring clip 128 is another example of a mechanical fastener that is suitable for releasably securing a modular valve assembly to an endoscopic device. In this example, the outer surface of the handpiece 12 can be considered a mating fastener that cooperates with the spring clip 128 to releasably hold the valve assembly 120 in place on the endoscopic device. The details of construction of the modular valve assembly 120 can be varied widely, in accordance with any of the other valve assemblies described in this specification.

Figure 13 provides a sectional view of another modular valve assembly 140. The modular valve assembly 140 includes a housing 142 that supports an inlet port 144 and an outlet port 146. A valve element 148 is slidably received in a cylinder defined by the housing 142, and the valve element 148 defines an annular recess 150. The annular recess 150 completely encircles the valve element 148, and thereby provides an interconnecting flow path between the inlet port 144 and the outlet port 146 when the recess 150 is aligned with the ports 144, 146. The valve element 148 is biased to the upper position shown in Figure 13 by a spring 152. The valve assembly 140 includes an actuator 156 that can be pressed downwardly by a finger of the user. A latch 154 is interposed between the actuator 156 and the valve element 148, and the latch 154 operates to hold the valve element 148 in a selected position.

In use, the inlet port 144 is coupled to a source of irrigation fluid and the outlet port 146 is coupled to the irrigation port of an endoscopic device. In the position shown in Figure 13, the recess 150 is out of alignment with the inlet and outlet ports 144, 146, and no irrigation fluid is passed to the outlet port 146. When the user presses the actuator 156 downwardly in the view of Figure 13, the recess 150 comes into alignment with the inlet and outlet ports 144, 146, thereby permitting irrigation liquid to flow to the endoscopic device. Further downward movement of the actuator 156 causes the latch 154 to hold the valve element 148 in a position in which the recess 150 is aligned with the inlet and outlet ports 144, 146. Once the latch 154 is engaged, the user can take his or her hand off of the actuator 156, and high volume flow of irrigation fluid is maintained from the inlet port 144 to the outlet port 146.

In order to stop the flow of irrigation fluid, the user again depresses the actuator 156, thereby causing the latch 154 to release the valve element 148 to move upwardly, back to the position of Figure 13.

The valve assembly 140 allows the user to modulate the flow of irrigation fluid as described above as he or she gradually depresses the actuator 156. The latch 154 also allows the user to latch the valve in the open position, until it is released by the user.

Many alternative structures can be used for the latch 154. For example, the latch 154 can be constructed like the latch mechanism conventionally used with retractable ballpoint pens. Such latch mechanisms respond to first depression of the actuator by latching the latched element down, and they respond to a next depression of the actuator by allowing the latched element to move upwardly. This is only one example, and many alternatives are possible.

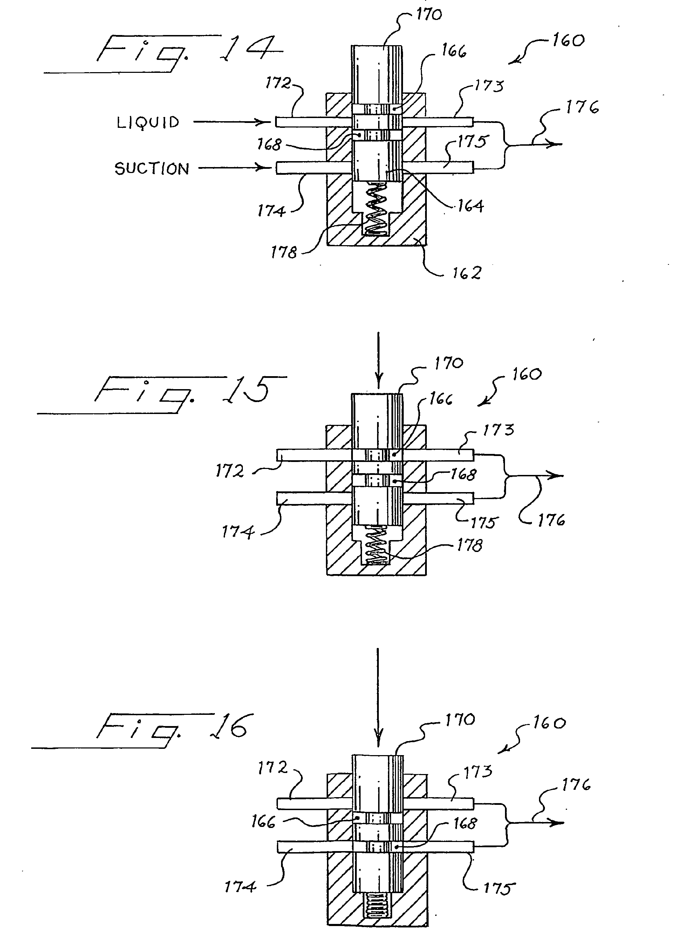

Figures 14, 15 and 16 provide three views of another modular valve assembly 160 that can be used as described above. As best shown in Figure 14, the modular valve assembly 160 includes a housing 162 that supports a valve element 164 for sliding movement. The valve element 164 defines two spaced, annular recesses 166, 168, and the upper end of the valve element 164 forms an actuator 170. The valve element 164 is biased to the upper position shown in Figure 14 by a spring 178.

The housing 162 supports first and second inlet ports 172, 174 and aligned tubes 173, 175 that are connected to an outlet port 176. The first inlet port 172 in use is connected to a liquid source, such as a source of irrigation fluid. The second inlet port 174 in use is connected to a suction source, such as a partial vacuum. The outlet port 176 in use is connected to an irrigation port of an endoscopic device. Check valves, not shown, may be used to prevent flow from the tube 173 to the tube 175 and vice-versa.

In the rest position of Figure 14, the valve element 164 isolates both the first and second inlet ports 172, 174 from the outlet port 176. This is because the first inlet port 172 is out of alignment with the first recess 166, and the second inlet port 174 is out of alignment with the second recess 168.

Figure 15 shows the valve assembly 160 in a second position, in which the user has depressed the actuator 170, thereby compressing the spring 178 and bringing the first recess 166 into alignment with the first inlet port 172 and the tube 173. In this position, irrigation fluid from the liquid source is passed by the assembly 160 to the outlet port 176.

As shown in Figure 16, when the actuator 170 is further depressed, the first recess 166 is moved out of alignment with the first inlet port 172, and the second recess 168 is moved into alignment with the second inlet port 174. In this position, the valve assembly 160 allows suction from the suction source to pass via the second inlet port 174 and the second tube 175 to the outlet port 176.

The modular valve assembly 160 of Figures 14 through 16 is intended to be removably attached to the handpiece of an endoscopic device, all as described above. Any of the mechanisms described above for releasably securing the valve assembly to the handpiece can be used. The valve assembly 160 provides all of the functions described above regarding the valving of irrigation fluid from the liquid source to the outlet port 176. In addition, the valve assembly 160 allows the physician efficiently and easily to introduce suction to the endoscopic device by moving the actuator 170 to the fully depressed position of Figure 16. Thus, a single valve assembly controls both the introduction of irrigation fluid and the application of suction to the irrigation port of the endoscopic device.

The valve assembly 160 utilizes a linear slide valve to implement the valving functions described above. It should of course be understood that this invention is not limited to such linear slide valves, and that the widest variety of valve mechanisms can be used to perform these valving functions.

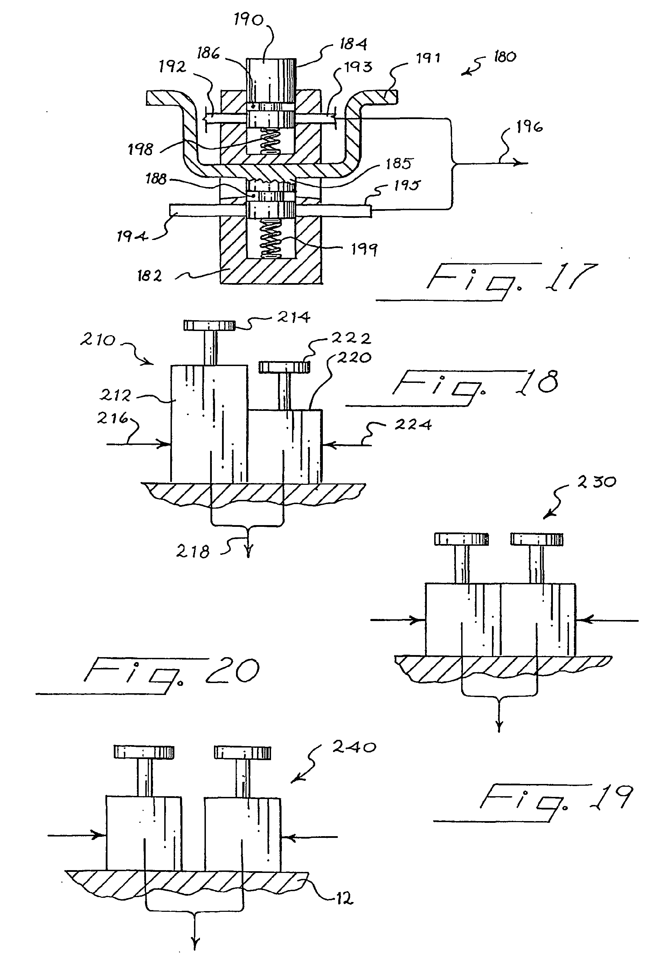

Figure 17 shows a sectional view of another modular valve assembly 180 also intended to be releasably secured to the handpiece of an endoscopic device as described above. The modular valve assembly 180 includes a housing 182 that supports first and second valve elements 184, 185. The first valve element 184 includes a first recess 186 and a first actuator 190. The first valve element 184 is biased to the upper position shown in Figure 17 by a spring 198. In this upper position the valve element 184 blocks the flow of liquid between a first inlet port 192 and a tube 193. As shown in Figure 17, the tube 193 is coupled to an outlet port 196, which may in turn be coupled to an irrigation port of an endoscopic device as described above (not shown). When the first actuator 190 is depressed to bring the first recess 186 into alignment with the first inlet port 192 and the first tube 193, irrigation fluid from a liquid source (not shown) passes from the first inlet port 192 to the outlet port 196.

The second valve element 185 defines a second recess 188 and is biased to an upper position as shown in Figure 17 by a second spring 199. The upper portion of the second valve element 185 is coupled to a second actuator 191. In this non-limiting example, the second actuator 191 is arranged so that the physician can reach it from any side of the valve assembly 180. This can be accomplished by forming the upper portion of the actuator 191 as a ring that encircles the housing 182. Alternatively, the actuator 191 may include a swivel, not shown, that allows the physician to rotate the upper portion of the actuator 191 to a desired angular position relative to the lower portion of the actuator 191 about an axis parallel to the sliding motion of the second valve element 185. In the rest position shown in Figure 17, the second valve element 185 blocks the flow of suction from a second inlet port 194 to the tube 195 (which is in turn coupled to the outlet port 196). When the user depresses the second actuator 191 to bring the second recess 188 into alignment with the second inlet port 194 and the second tube 195, suction is applied to the outlet port 196.

The modular valve assembly 180 is provided with adhesive straps, mechanical fasteners, spring clips or the like for releasably securing it to the handpiece of an endoscopic device (not shown). The modular valve assembly 180 allows the user to control the flow of irrigation fluid and the application of suction to the outlet port 196. In this case, the user moves his or her finger between the first and second actuators 190, 191 to provide irrigation fluid or suction to the outlet port 196, respectively.

Figure 18 shows another modular valve assembly 210 that performs all of the functions described above in conjunction with Figures 16 and 17. The modular valve assembly 210 includes a housing 212, 220 that supports two separate valves, each controlled by a respective actuator 214, 222. The actuator 214 controls the flow of irrigation fluid between a first inlet port 216 and an outlet port 218, and the second actuator 222 controls the introduction of suction from the second inlet port 224 to the outlet port 218. In this case the actuators 214, 222 and the associated valves are positioned in side-by-side relationship, but at differing elevations to assist the user in discriminating between the two actuators 214, 222.

The modular valve assembly 230 of Figure 19 is similar to the valve assembly 210, except that in this case the two actuators are positioned at the same elevation.

The modular valve assembly 240 of Figure 20 is similar to the modular valve assembly 230, but in this case the two valves are mounted some distance from one another on the handpiece 12. Figure 20 shows the manner in which a housing may include two or more spatially separated parts.

The modular valve assemblies of Figures 12 through 20 are all intended to be releasably mounted to an endoscopic device and to allow the user to control the flow of at least irrigation fluid to the irrigation port of the endoscopic device. The modular valve assemblies of Figures 14 through 20 additionally allow the user to control the application of suction to the irrigation port. The valve assemblies of Figures 14 through 20 are used in the same manner as the valves described above, except that the first inlet port 172, 192, 212 is connected to a source of irrigation fluid and the second inlet port 174, 194, 224 is connected to a source of suction prior to the surgical procedure. This can be done either before or after the modular valve assembly 160, 180, 210, 230, 240 is releasably mounted to the handpiece of the endoscopic device.

It should be apparent from the foregoing description that the improved modular valve assembly of this invention provides the important advantage that little or no modification is required to a conventional endoscope, yet the physician using the endoscope is provided with improved control over the flow of irrigation fluid. In particular, the physician can use direct finger pressure to modulate the flow of irrigation fluid as desired, while still leaving one hand free for surgical procedures. In this way, the need for a trained surgical nurse is reduced, and the physician's control over irrigation fluid flow is improved. The valve assembly described above is well suited for use with a wide variety of endoscopes including modern, small endoscopes that are too small for built-in valves.

Of course, it should be understood that a wide range of changes and modifications can be made to the preferred embodiments described above. For example, the valve of the valve assembly can take any suitable form, and it is not limited to the specific examples described above. The motion used to open or close the valve 36 can be varied as appropriate for the application, and it can include a lifting motion, a depressing motion, a sliding motion parallel to the length of the handpiece, or a rotating motion as desired. As a further alternative, the valve may be implemented as an element that pinches a resilient tube to slow or block flow through the tube. Thus, the valve can be implemented as a one-piece or a multiple-piece system having sliding, hinged, rotating or other motions.

Similarly, the mechanical fasteners that releasably hold the valve assembly in place on the handpiece of the endoscope can take any suitable form, and such fasteners are not limited to the adhesives, straps, snap-lock studs, and recesses described above. Many other mechanical fasteners can be adapted for use with this invention, as for example linear or rotary guides (including, e.g., dovetail guides or bayonet sockets) and various types of resilient or bendable elements that releasably hold the valve assembly in place.

As used herein, the term "position" is intended broadly to encompass a range of positions. Thus, the valve may block fluid flow between the inlet and outlet ports in a range of blocking positions and the valve may allow fluid to flow from the inlet port to the outlet port in a range of opened positions. The valve may be configured as an on/off valve or as a modulating valve.

The term "handpiece" is intended broadly to refer to the part of an endoscope that carries the eyepiece and is held by the user, whether referred to as the handpiece, the bridge, or by some other term by the manufacturer of the endoscope.

The term "housing" is intended broadly to include one-part housings as well as housings having two or more parts that may be physically integrated with one another or spatially separated from one another.

The term "valve" is intended broadly to encompass valves having one or more moveable valve elements controlling the flow of one or more fluids.

The term "inlet port" is intended broadly to refer to a port that is connected either to a fluid source or to a suction source.

Also, any suitable structure can be used for pressurizing the irrigation liquid, including simple gravity feeds in some examples.

The foregoing detailed description has discussed only a few of the many forms that this invention can take. This detailed description is therefore intended by way of illustration and not by way of limitation. It is only the following claims, including all equivalents, that are intended to define the scope of this invention.

## Claims

1. A modular endoscope valve assembly (30) adapted to be releasably mounted to an exterior surface (20) of an endoscope (10) having a handpiece (12) and an irrigation port (18), the modular endoscope valve assembly (30) comprising:
a housing (50) comprising an inlet port (32), an outlet port (34), and a mounting surface (52); and
a valve (36) carried by the housing (50) and coupled between the inlet port (32) and the outlet port (34), the valve (36) comprising a manually-controlled actuator (38) movable by a user between a first position, in which the valve (36) blocks flow between the inlet port (32) and the outlet port (34), and a second position, in which the valve (36) allows flow between the inlet port (32) and the outlet port (34), **characterised in that** the assembly further comprises ;
a strap (42) carried by the housing (50) and operative to releasably secure the housing (50) to the handpiece (12) in such a way that the mounting surface (52) of the housing (50) is releasably held in place on the exterior surface (20) by the strap (42); and
**in that** the outlet port (34) is adapted to be releasably connected to the irrigation port (18) of the endoscope (10).

2. The invention of Claim 1 further comprising a pressure-sensitive adhesive (40) carried by the mounting surface (52) and operative to releasably secure the housing (50) to the endoscope (10).

3. The apparatus as claimed in Claim 1 wherein the actuator (38) is mounted to slide linearly between the first and second positions.

4. The apparatus as claimed in Claim 1 wherein the actuator (38) is mounted to rotate between the first and second positions.

5. An apparatus as claimed in Claim 1 wherein the endoscope (10) comprises a ureteroscope.

6. The apparatus as claimed in Claim 1 wherein the outlet port (34) comprises a contrast-introduction port (48).

7. The apparatus as claimed in Claim 1 further comprising a latch (154) coupled with the valve (148) and operative to releasably hold the valve (148) in a selected state in response to a control input by the user.

8. The apparatus of Claim 7 wherein the latch (154) is coupled to the actuator (156) and operative to hold the valve (148) in a state that allows flow between the inlet port (144) and the outlet port (146) in response to user manipulation of the actuator (156).

9. The apparatus as claimed in Claim 1 wherein the housing (162) further comprises a second inlet port (174), wherein the valve (164) blocks flow between the second inlet port (174) and the outlet port (176) in the first and second positions, and wherein the actuator (170) is movable by the user to a third position, in which the valve (164) allows flow between the second inlet port (174) and the outlet port (176) while blocking flow between the first-mentioned inlet port (172) and the outlet port (176).

10. The apparatus of Claim 9 wherein the first-mentioned inlet port (172) is connected to a fluid source, and wherein the second inlet port (174) is connected to a suction source.

11. The apparatus as claimed in Claim 1 further comprising:
a second inlet port (194) included in the housing (182); and
a second valve (185) carried by the housing (182) and coupled between the second inlet port (194) and the outlet port (196), the second valve (185) comprising a manually controlled second actuator (191) movable by the user between a third position, in which the second valve (185) blocks flow between the second inlet port (194) and the outlet port (196), and a fourth position, in which the second valve (185) allows flow between the second inlet port (194) and the outlet port (196).

12. The apparatus as claimed in Claim 11 wherein the first-mentioned inlet port (192) is connected to a liquid source, and wherein the second inlet port (194) is connected to a suction source.

13. A method for enhancing control efficiency of a medical endoscope (10), the method comprising:
(a) providing a medical endoscope (10) comprising an exterior surface (20) and an irrigation port (18);
(b) providing a modular endoscope valve assembly (30) comprising:
a housing comprising an inlet port (32), an outlet port (34), and a mounting surface (52);
a valve (36) carried by the housing (50) and coupled between the inlet port (32) and the outlet port (34), the valve (36) comprising a manually-controlled actuator (38) movable by a user between a first position, in which the valve (36) blocks flow between the inlet port (32) and the outlet port (34), and a second position, in which the valve (36) allows flow between the inlet port (32) and the outlet port (34); and
a strap (42) carried by the housing (50);
(c) releasably securing the valve assembly (30) to the exterior surface (20) of the endoscope (10) with the strap (42) in such a way that the mounting surface (52) of the housing (50) is releasably held in place on the exterior surface (20) by the strap (42);
(d) removably coupling the outlet port (34) of the valve assembly (30) to the irrigation port (18) of the endoscope (10); and then
(e) removing the valve assembly (30) from the exterior surface (20) of the endoscope (10) after a surgical procedure.

14. The method of Claim 13 wherein the valve assembly (140) provided in (b) further comprises a latch (154) coupled with the valve (148) and operative to releasably hold the valve (148) in a selected state in response to a control input by the user.

15. The method of Claim 14 wherein the latch (154) is coupled to the actuator (156) and operative to hold the valve (148) in a state that allows flow between the inlet port (144) and the outlet port (146) in response to user manipulation of the actuator (156).

16. The method of Claim 13 wherein the housing (162) provided in (b) further comprises a second inlet port (174), wherein the valve (164) blocks flow between the second inlet port (174) and the outlet port (176) in the first and second positions, and wherein the actuator (170) is movable by the user to a third position, in which the valve (164) allows flow between the second inlet port (174) and the outlet port (176) while blocking flow between the first-mentioned inlet port (172) and the outlet port (176).

17. The method of Claim 16 further comprising:
(f) connecting the first-mentioned inlet port (172) to a liquid source prior to (e); and
(g) connecting the second inlet port (174) to a suction source prior to (e).

18. The method of Claim 13 wherein the housing (182) provided in (b) further comprises a second inlet port (194), and wherein the valve assembly (180) provided in (b) further comprises a second valve (185) carried by the housing (182) and coupled between the second inlet port (194) and the outlet port (196), the second valve (185) comprising a manually controlled second actuator (191) movable by the user between a third position, in which the second valve (185) blocks flow between the second inlet port (194) and the outlet port (196), and a fourth position, in which the second valve (185) allows flow between the second inlet port (194) and the outlet port (196).

19. The method of Claim 18 further comprising:
(f) connecting the first-mentioned inlet port (192) to a liquid source prior to (e); and
(g) connecting the second inlet port (194) to a suction source prior to (e).

20. The method of claim 13 wherein the modular endoscope valve assembly (30) comprises a pressure-sensitive adhesive (40) carried by the mounting surface (52), and wherein (c) comprises releasably securing the valve assembly (30) to the exterior surface (20) of the endoscope (10) with the adhesive (40).

## Patentansprüche

1. Ventilanordnung (30) für ein modulares Endoskop, welche Ventilanordnung so ausgelegt ist, dass sie lösbar an einer Außenfläche (20) eines Endoskops (10), welches ein Handstück (12) sowie eine Spülungsöffnung (18) aufweist, befestigt wird, wobei die Ventilanordnung (30) für das modulare Endoskop Folgendes aufweist:
ein Gehäuse (50), welches eine Einlassöffnung (32), eine Auslassöffnung (34) und eine Befestigungsfläche (52) umfasst; und
ein Ventil (36), welches von dem Gehäuse (50) gestützt wird und zwischen der Einlassöffnung (32) und der Auslassöffnung (34) gekoppelt ist, wobei das Ventil (36) ein manuell geregeltes Betätigungsglied (38) aufweist, welches von einem Benutzer zwischen einer ersten Position, in welcher das Ventil (36) einen Fluss zwischen der Einlassöffnung (32) und der Auslassöffnung (34) blockiert, und einer zweiten Position, in welcher das Ventil (36) den Fluss zwischen der Einlassöffnung (32) und der Auslassöffnung (34) zulässt, verschiebbar bzw. bewegbar ist, **dadurch gekennzeichnet, dass** die Anordnung des Weiteren Folgendes aufweist:
ein Band (42), welches von dem Gehäuse (50) getragen wird und funktionsbereit ist, um das Gehäuse (50) lösbar an dem Handstück (12) derart zu befestigen, dass die Befestigungsfläche (52) des Gehäuses (50) mit Hilfe des Bandes (42) lösbar auf der Außenfläche (20) in Position gehalten wird; und
**dadurch gekennzeichnet, dass** die Auslassöffnung (34) derart ausgelegt ist, dass sie lösbar an die Spülungsöffnung (18) des Endoskops (10) angeschlossen wird.

2. Erfindung nach Anspruch 1, welche des Weiteren ein druckempfindliches Haftmittel (40) aufweist, welches auf die Befestigungsfläche (52) aufgetragen ist und funktionsbereit ist, um das Gehäuse (50) lösbar an dem Endoskop (10) zu befestigen.

3. vorrichtung nach Anspruch 1, **dadurch gekennzeichnet, dass** das Betätigungsglied (38) derart angebracht ist, dass es linear zwischen der ersten und zweiten Position gleitet.

4. Vorrichtung nach Anspruch 1, **dadurch gekennzeichnet, dass** das Betätigungsglied (38) derart angebracht ist, dass es sich zwischen der ersten und zweiten Position dreht.

5. vorrichtung nach Anspruch 1, **dadurch gekennzeichnet, dass** das Endoskop (10) ein Ureteroskop aufweist.

6. Vorrichtung nach Anspruch 1, **dadurch gekennzeichnet, dass** die Auslassöffnung (34) eine Kontrastmittel-Einführöffnung (48) aufweist.

7. Vorrichtung nach Anspruch 1, welche des Weiteren eine Sperre (154) aufweist, welche mit dem Ventil (148) verbunden ist und funktionsbereit ist, um das Ventil (148) ansprechend auf eine Steuereingabe durch den Benutzer in einem gewählten zustand zu halten.

8. Vorrichtung nach Anspruch 7, **dadurch gekennzeichnet, dass** die Sperre (154) mit dem Betätigungsglied (156) gekoppelt ist und funktionsbereit ist, um das ventil (148) ansprechend auf eine Betätigung bzw. Bedienung des Betätigungsgliedes (156) durch den Benutzer in einem Zustand zu halten, welcher den Fluss zwischen der Einlass- öffnung (144) und der Auslassöffnung (146) zulässt.

9. Vorrichtung nach Anspruch 1, **dadurch gekennzeichnet, dass** das Gehäuse (162) des Weiteren eine zweite Einlassöffnung (174) aufweist, wobei das Ventil (164) den Fluss zwischen der zweiten Einlassöffnung (174) und der Auslassöffnung (176) in der ersten und zweiten Position blockiert, und wobei das Betätigungsglied (170) mit Hilfe des Benutzers in eine dritte Position verschiebbar bzw, bewegbar ist, in welcher das Ventil (164) den Fluss zwischen der zweiten Einlassöffnung (174) und der Auslassöffnung (176) zulässt, während ein Fluss zwischen der erstgenannten Einlassöffnung (172) und der Auslassöffnung (176) blockiert wird.

10. Vorrichtung nach Anspruch 9, **dadurch gekennzeichnet, dass** die erstgenannte Einlassöffnung (172) mit einer Fluidquelle verbunden ist, und dass die zweite Einlassöffnung (174) mit einer Ansaugquelle verbunden ist.

11. Vorrichtung nach Anspruch 1, welche des Weiteren Folgendes aufweist:
eine zweite Einlassöffnung (194), welche in dem Gehäuse (182) enthalten ist; und
ein zweites Ventil (185), welches von dem Gehäuse (182) gestützt wird und zwischen der zweiten Einlassöffnung (194) und der Auslassöffnung (196) angeschlossen ist, wobei das zweite Ventil (185) ein manuell geregeltes zweites Betätigungsglied (191) aufweist, welches von dem Benutzer zwischen einer dritten Position, in welcher das zweite Ventil (185) den Fluss zwischen der zweiten Einlassöffnung (194) und der Auslassöffnung (196) blockiert, und einer vierten Position, in welcher das zweite Ventil (185) den Fluss zwischen der zweiten Einlassöffnung (194) und der Auslassöffnung (196) zulässt, bewegbar bzw. verschiebbar ist.

12. Vorrichtung nach Anspruch 11, **dadurch gekennzeichnet, dass** die erstgenannte Einlassöffnung (192) mit einer Flüssigkeitsquelle verbunden ist, und dass die zweite Einlassöffnung (194) mit einer Ansaugquelle verbunden ist.

13. Verfahren zur Verbesserung der Steuereffizienz eines medizinischen Endoskops (10), wobei das Verfahren die folgenden Schritte aufweist:
(a) Bereitstellung eines medizinischen Endoskops (10), welches eine Außenfläche (20) und eine Spülungsöffnung (18) aufweist;
(b) Bereitstellung einer Ventilanordnung (30) für ein modulares Endoskop, welche Ventilanordnung Folgendes aufweist:
ein Gehäuse mit einer Einlassöffnung (32), einer Auslassöffnung (34) und einer Befestigungsfläche (52);
ein Ventil (36), welches von dem Gehäuse (50) gestützt wird und welches zwischen der Einlassöffnung (32) und der Auslassöffnung (34) gekoppelt ist, wobei das Ventil (36) ein manuell geregeltes Bedienelement (38) aufweist, welches von einem Benutzer zwischen einer ersten Position, in welcher das Ventil (36) den Fluss zwischen der Einlassöffnung (32) und der Auslassöffnung (34) blockiert, und einer zweiten Position, in welcher das Ventil (36) den Fluss zwiscen der Einlassöffnung (32) und der Auslassöffnung (34) zulässt, bewegbar ist; und
ein Band (42), welches von dem Gehäuse (50) getragen wird;
(c) lösbare Befestigung der Ventilanordnung (30) an der Außenfläche (20) des Endoskops (10) mit Hilfe des Bandes (42) derart, dass die Befestigungsfläche (52) des Gehäuses (50) mit Hilfe des Bandes (42) lösbar auf der Außenfläche (20) in Position gehalten wird;
(d) lösbare Kopplung der Auslassöffnung (34) der Ventilanordnung (30) an die Spülungsöffnung (18) des Endoskops (10); und anschließende
(e) Entfernung der Ventilanordnung (30) von der Außenfläche (20) des Endoskops (10) nach einem chirurgischen Eingriff.

14. Verfahren nach Anspruch 13, **dadurch gekennzeichnet, dass** die in (b) bereitgestellte Ventilanordnung (140) des Weiteren eine Sperre (154) aufweist, welche mit dem Ventil (148) gekoppelt ist und funktionsbereit ist, um das Ventil (148) ansprechend auf eine Steuereingabe durch den Benutzer in einem ausgewählten Zustand zu halten.

15. Verfahren nach Anspruch 14, **dadurch gekennzeichnet, dass** die Sperre (154) mit dem Betätigungsglied (156) gekoppelt ist und funktionsbereit ist, um das Ventil (148) in einem Zustand zu halten, welcher den Fluss zwischen der Einlassöffnung (144) und der Auslassöffnung (146) ansprechend auf eine Betätigung bzw. Bedienung des Betätigungsglieds (156) durch den Benutzer zulässt.

16. Verfahren nach Anspruch 13, **dadurch gekennzeichnet, dass** das in schritt (b) bereitgestellte Gehäuse (162) des Weiteren eine zweite Einlassöffnung (174) aufweist, wobei das Ventil (164) den Fluss zwischen der zweiten Einlassöffnung (174) und der Auslassöffnung (176) in der ersten und zweiten Position blockiert, und wobei das Betätigungsglied (170) vom Benutzer in eine dritte Position bewegbar bzw. verschiebbar ist, in welcher das Ventil (164) den Fluss zwischen der zweiten Einlassöffnung (174) und der Auslassöffnung (176) zulässt, während der Fluss zwischen der erstgenannten Einlassöffnung (172) und der Auslassöffnung (176) blockiert wird.

17. Verfahren nach Anspruch 16, welches des Weiteren die folgenden Schritte aufweist:
(f) Verbindung der erstgenannten Einlassöffnung (172) mit einer Flüssigkeitsquelle vor Schritt (e); und
(g) Verbindung der zweiten Einlassöffnung (174) mit einer Ansaugquelle vor Schritt (e).

18. Verfahren nach Anspruch 13, **dadurch gekennzeichnet, dass** das in Schritt (b) bereitgestellte Gehäuse (182) des Weiteren eine zweite Einlassöffnung (194) aufweist, und dass die in Schritt (b) bereitgestellte Ventilanordnung (180) des Weiteren ein zweites Ventil (185) aufweist, welches von dem Gehäuse (182) getragen wird und zwischen der zweiten Einlassöffnung (194) und der Auslassöffnung (196) gekoppelt ist, wobei das zweite Ventil (185) ein manuell geregeltes zweites Betätigungsglied (191) aufweist, welches von dem Benutzer zwischen einer dritten Position, in welcher das zweite Ventil (185) den Fluss zwischen der zweiten Einlassöffnung (194) und der Auslassöffnung (196) blockiert, und einer vierten Position, in welcher das zweite Ventil (185) den Fluss zwischen der zweiten Einlassöffnung (194) und der Auslassöffnung (196) zulässt, bewegbar bzw. verschiebbar ist.

19. Verfahren nach Anspruch 18, welches des Weiteren die folgenden Schritte aufweist:
(f) Verbindung der erstgenannten Einlassöffnung (192) mit einer Flüssigkeitsquelle vor Schritt (e); und
(g) Verbindung der zweiten Einlassöffnung (194) mit einer Ansaugquelle vor Schritt (e).

20. Verfahren nach Anspruch 13, **dadurch gekennzeichnet, dass** die Ventilanordnung (30) für das modulare Endoskop ein druckempfindliches Haftmittel (40) aufweist, welches auf die Befestigungsfläche (52) aufgetragen ist, und dass Schritt (c) die lösbare Befestigung der Ventilanordnung (30) an der Außenfläche (20) des Endoskops (10) mit Hilfe des Haftmittels (40) aufweist.

## Revendications

1. Ensemble de valve d'endoscope modulaire (30) apte à être monté amoviblement sur une surface extérieure (20) d'un endoscope (10) ayant une pièce à main (12) et un orifice d'irrigation (18), l'ensemble de valve d'endoscope modulaire (30) comprenant:
un boîtier (50) comprenant un orifice d'entrée (32), un orifice de sortie (34) et une surface de montage (52); et
une valve (36) supportée par le boîtier (50) et couplée entre l'orifice d'entrée (32) et l'orifice de sortie (34), la valve (36) comprenant un actionneur à commande manuelle (38) déplaçable par un utilisateur entre une première position dans laquelle la valve (36) bloque l'écoulement entre l'orifice d'entrée (32) et l'orifice de sortie (34) et une deuxième position dans laquelle la valve (36) permet l'écoulement entre l'orifice d'entrée (32) et l'orifice de sortie (34), **caractérisé en ce que** l'ensemble comprend en outre:
une bande (42) supportée par le boîtier (50) et apte à fixer amoviblement le boîtier (50) à la pièce à main (12) de telle manière que la surface de montage (52) du boîtier (50) est retenue relâchablement en place sur la surface extérieure (20) par la bande (42); et
**en ce que** l'orifice de sortie (34) est apte à être relié amoviblement à l'orifice d'irrigation (18) de l'endoscope (10).

2. Appareil selon la revendication 1, comprenant en outre un adhésif sensible à la pression (40) supporté par la surface de montage (52) et apte à fixer amoviblement le boîtier (50) à l'endoscope (10).

3. Appareil selon la revendication 1, où l'actionneur (38) est monté pour coulisser linéairement entre les première et seconde positions.

4. Appareil selon la revendication 1, où l'actionneur (38) est monté pour tourner entre les première et seconde positions.

5. Appareil selon la revendication 1, où l'endoscope (10) comprend un hystéroscope.

6. Appareil selon la revendication 1, où l'orifice de sortie (34) comprend un orifice d'introduction de produit de contraste (48).

7. Appareil selon la revendication 1, comprenant en outre un verrouillage (154) couplé avec la valve (148) et apte à tenir relâchablement la valve (148) dans un état sélectionné en réponse à une entrée de commande par l'utilisateur.

8. Appareil selon la revendication 7, où le verrouillage (154) est couplé à l'actionneur (156) et est apte à maintenir la valve (148) dans un état qui permet un écoulement entre l'orifice d'entrée (144) et l'orifice de sortie (146) en réponse à la manipulation par l'utilisateur de l'actionneur (156).

9. Appareil selon la revendication 1, où le boîtier (162) comprend en outre un deuxième orifice d'entrée (174), où la valve (164) bloque l'écoulement entre la deuxième portion d'entrée (174) et l'orifice de sortie (176) dans les première et deuxième positions, et où l'actionneur (166) est déplaçable par l'utilisateur à une troisième position dans laquelle la valve (164) permet l'écoulement entre le deuxième orifice d'entrée (174) et l'orifice de sortie (176) tout en bloquant l'écoulement entre l'orifice d'entrée (172) mentionné en premier et l'orifice de sortie (176).

10. Appareil selon la revendication 9, où l'orifice d'entrée (172) mentionné en premier est relié à une source de fluide, et où le deuxième orifice d'entrée (174) est relié à une source d'aspiration.

11. Appareil selon la revendication 1, comprenant en outre:
un deuxième orifice d'entrée (194) inclus dans le boîtier (182); et
une deuxième valve (185) portée par le boîtier (182) et couplée entre le deuxième orifice d'entrée (194) et l'orifice de sortie (196), la deuxième valve (185) comprenant un deuxième actionneur (191) à commande manuelle déplaçable par l'utilisateur entre une troisième position dans laquelle la deuxième valve (185) bloque l'écoulement entre le deuxième orifice d'entrée (194) et l'orifice de sortie (196) et une quatrième position dans laquelle la deuxième valve (185) permet l'écoulement entre le deuxième orifice d'entrée (194) et l'orifice de sortie (196).

12. Appareil selon la revendication 11, où l'orifice d'entrée (192) mentionné en premier est relié à une source de liquide, et où le deuxième orifice d'entrée (194) est relié à une source d'aspiration.

13. Procédé pour augmenter l'efficacité de contrôle d'un endoscope médical (10), le procédé comprenant:
(a) réaliser un endoscope médical (10) comprenant une surface extérieure (20) et un orifice d'irrigation (18);
(b) réaliser un ensemble de valve d'endoscope modulaire (30) comprenant:
un boîtier comprenant un orifice d'entrée (32), un orifice de sortie (34) et une surface de montage (52);
une valve (36) supportée par le boîtier (50) et couplée entre l'orifice d'entrée (32) et l'orifice de sortie (34), la valve (36) comprenant un actionneur (38) à commande manuelle déplaçable par un utilisateur entre une première position dans laquelle la valve (36) bloque l'écoulement entre l'orifice d'entrée (32) et l'orifice de sortie (34), et une deuxième position dans laquelle la valve (36) permet l'écoulement entre l'orifice d'entrée (32) et l'orifice de sortie (34); et
une bande (42) portée par le boîtier (50);
(c) fixer amoviblement l'ensemble de valve (30) à la surface extérieure (20) de l'endoscope (10) avec la bande (42) de telle manière que la surface de montage (52) du boîtier (50) est retenue relâchablement en place sur la surface extérieure (20) par la bande (42);
(d) coupler amoviblement l'orifice de sortie (34) de l'ensemble de valve (30) à l'orifice d'irrigation (18) de l'endoscope (10); et ensuite
(e) retirer l'ensemble de valve (30) de la surface extérieure (20) de l'endoscope (10) après l'intervention chirurgicale.

14. Procédé selon la revendication 13, où l'ensemble de valve (140) réalisé dans (b) comprend en outre un verrouillage (154) couplé à la valve (148) et apte à tenir relâchablement la valve (148) dans un état sélectionné en réponse à une entrée de commande par l'utilisateur.

15. Procédé selon la revendication 14, où le verrouillage (154) est couplé à l'actionneur (156) et est apte à tenir la valve (148) dans un état qui permet l'écoulement entre l'orifice d'entrée (144) et l'orifice de sortie (146) en réponse à la manipulation par l'utilisateur de l'actionneur (156).

16. Procédé selon la revendication 13, dans lequel le boîtier (162) réalisé dans (b) comprend en outre un deuxième orifice d'entrée (174), où la valve (164) bloque l'écoulement entre le deuxième orifice d'entrée (174) et l'orifice de sortie (176) dans les première et deuxième positions, et où l'actionneur (170) est déplaçable par l'utilisateur à une troisième position dans laquelle la valve (164) permet l'écoulement entre le deuxième orifice d'entrée (174) et l'orifice de sortie (176) tout en bloquant l'écoulement entre l'orifice d'entrée (172) mentionné en premier et l'orifice de sortie (176).

17. Procédé selon la revendication 16, comprenant en outre:
(f) relier l'orifice d'entrée (172) mentionné en premier à une source de liquide avant (e); et
(g) relier le deuxième orifice d'entrée (174) à une source d'aspiration avant (e).

18. Procédé selon la revendication 13, dans lequel le boîtier (182) réalisé dans (b) comprend en outre un deuxième orifice d'entrée (194), et où l'ensemble de valve (180) réalisé dans (b) comprend en outre une deuxième valve (185) supportée par le boîtier (182) et couplée entre le deuxième orifice d'entrée (194) et l'orifice de sortie (196), la deuxième valve (185) comprenant un deuxième actionneur à commande manuelle (191) déplaçable par l'utilisateur entre une troisième position dans laquelle la deuxième valve (185) bloque l'écoulement entre le deuxième orifice d'entrée (194) et l'orifice de sortie (196), et une quatrième position dans laquelle la deuxième valve (185) permet l'écoulement entre le deuxième orifice d'entrée (194) et l'orifice de sortie (196).

19. Procédé selon la revendication 18, comprenant en outre:
(f) relier l'orifice d'entrée (192) mentionné en premier à une source de liquide avant (e); et
(g) relier le deuxième orifice d'entrée (194) à une source d'aspiration avant (e).

20. Procédé selon la revendication 13, où l'ensemble de valve d'endoscope modulaire (30) comprend un adhésif (40) réagissant à la pression supporté par la surface de montage (52), et où (c) comprend la fixation amovible de l'ensemble de valve (30) à la surface extérieure (20) de l'endoscope (10) avec l'adhésif (40).
